# EUROPEAN PATENT APPLICATION

(11) **EP 4 299 059 A1**
(43) Date of publication of application: **03.01.2024**
(21) Application number: 22382628.0
(22) Date of filing: 01.07.2022
(51) Int. Cl.: A61K 31/05, A61K 31/455, A61K 31/7072, A61P 27/16, A61K 9/00

(54) **COMPOSITION FOR THE TREATMENT OF TINNITUS**

(71) Applicant: Labelic Analysis, S.L., 08036 Barcelona (ES)
(72) Inventor: AGUT SÁNCHEZ, Julián, 08197 SANT CUGAT DEL VALLÈS (ES); MARTÍNEZ-MONCHE ZARAGOZA, Gonzalo, 08006 BARCELONA (ES)
(74) Representative: Sugrañes, S.L.P.

(57) **Abstract**

The present invention refers to a composition for the treatment of tinnitus. It refers to a ternary combination of nicotinamide, uridine monophosphate and trans-resveratrol. It refers also to a composition, pharmaceutical or nutraceutical, which comprises said active compounds. It relates also to said combination and composition for the treatment of tinnitus and/or hearing loss disorders, and as neuroprotective.

## Description

### Field of the invention

The present invention relates to the field of compositions for the treatment of tinnitus and/or hearing loss disorders.

### Background art

Tinnitus, derived from the Latin verb *tinnire* (to ring), is a term that describes the conscious perception of an auditory sensation in absence of a corresponding external stimulus.

Tinnitus may be subjective, when it is only heard by the subject alone, or objective, when sometimes it can be heard by an observer and is caused by an internal bodily vibration or noise. It can sometimes be a rhythmical or pulsatile sound. It can be constant or intermittent. It can be localised to one or both ears, or centrally within the head. It may be categorised also as primary or secondary, wherein the former is idiopathic and may or may not be associated with sensorineural hearing loss, and the latter is associated with a specific underlying cause, as disclosed in A. A. Esmaili, A review of tinnitus, Aus. J. Gen. Pract., 2018, 47(4), 205-208.

Tinnitus is often considered idiopathic, although age-related hearing loss (presbyacusis) and noise exposure remain the most common causes, as disclosed in Wu et al., Approach to tinnitus management, Can. Fam. Phys., 2018, 64, 491-495.

According to Haider et a/., Pathophysiology of Subjective Tinnitus: Triggers and Maintenance, Front Neurosci., 2018, 12, 866, tinnitus pathophysiology is complex and multifactorial, involving the auditory and nonauditory systems, and tinnitus engages multiple active dynamic and overlapping networks.

The heterogeneity of tinnitus experience is substantial and has triggered both basic science and treatment research.

As disclosed in Baguley et a/., Tinnitus, The Lancet, 2018, 382, 1600-1607, the prevalence of troublesome tinnitus increases with age to 70 years. Tinnitus is a common medical symptom that can be debilitating, wherein risk factors include hearing loss, ototoxic medication, head injury, and depression. Tinnitus can greatly affect a patient's physical and psychological quality of life. In particular, tinnitus can disturb one's day-to-day life in a number of ways including distress and annoyance, disruption of sleep, anxiety and depression, as disclosed in a review published by the Agency for Healthcare Research and Quality (2012).

Due to the absence of an effective treatment of tinnitus, different approaches have been disclosed to reduce the consequences of said disorder. As disclosed in Baguley *et a*/*., op. cit.,* standard care includes sound therapy (either hearing aids or sound generators); in other cases, patients use complementary and alternative medicine; brain magnetic stimulation has been investigated to reduce neuronal activity; and drugs from several broad categories have been tested for effect on tinnitus.

Traditional Chinese medicine compositions administered orally have been disclosed for the treatment of tinnitus in, for example, CN-A-113476544, CN-A-112755115, and CN-A-112316063, as well as compositions in the form of ear drops in, for example, CN-A-111840403.

Vegetable extracts have also been disclosed to be used for the treatment of tinnitus. For example: *Vitis vinifera* leaf extract (WO-A-2021/166962), onion extract (US-A-2017/0007660), *Chenopodium* extract, quinin and *Conium maculatum* extract (DE-A-10200807868), blueberry extract (WO-A-2007/104566), *Moranda citrifolia* extract (WO-A-01/64231), *Ginkgo bilova* leaves extract (WO-A-99/47148), or St. John's wort extract (DE-A-19700788).

The diversity of approaches for the treatment of tinnitus, by means of the administration of drugs, is exemplified in international patent application WO-A-2009/132050, wherein it is disclosed a pharmaceutical composition for treating an otic disease or condition, such as tinnitus, among others, which comprises specific amounts of an immunomodulating agent, a polyoxyethylenepolyoxypropylene triblock copolymer and sterile water, wherein the immunomodulating agent is selected from anti-TNF agent, a calcineurin inhibitor, an IKK inhibitor, an interleukin inhibitor, a TNF-a converting enzyme inhibitor, or a toll-like receptor inhibitor. It further discloses that there are several treatments of tinnitus: lidocaine, administered intravenously, neurotransmitter reuptake inhibitors, such as nortriptyline, sertraline, and paroxetine, and benzodiazepines. In the Examples section, it further discloses clinical trials with different drugs (diazepam, neramexane, vestipitant/paroxitene) as a treatment of tinnitus.

In the review article Salvi et a/., Pharmacological treatments for tinnitus: new and old, Drugs Future, 2009, 34(5), 381-400, it is confirmed the variety of drugs that have been used with the target of treating tinnitus. The lack of drug therapies is due in part to a limited understanding of the biological basis of tinnitus, the lack of an accepted tinnitus nosology, the heterogeneity of the tinnitus population, the wide range of medical conditions that appear to cause tinnitus and the huge cost associated with developing drugs to specifically treat tinnitus. Consequently, drugs developed for other medical conditions have generally been evaluated to determine whether they can relieve tinnitus. Among such drugs, there are: acamprosate, caroverine, memantine, gacyclidine, alprazolam, vigabatrin, tiagabine, carbamazepine, gabapentin, paroxetine, trazodone, trimiramine, sertraline, misoprostol, atorvastatin, nimodipine, furosemide, scopolamide, sulpiride and melatonine.

Even recently, the use of additional drugs for the treatment of tinnitus has been disclosed: lecocloperastine (WO-A-2022/091118), cannabigerolic acid (WO-A-2022/082313), latanoprost (WO-A-2022/081297), sortilin antagonist (WO-A-2022/074119), oxytocin (WO-A-2021/042029), sazetidine (WO-A-2020/163588), and gaboxadol (WO-A-2018/144827).

Combinations of pharmaceutical active ingredients have also been disclosed in US-A-2020/0155483, such as ortriptyline and topiramate, nortriptyline and gabapentin, amitriptyline and topiramate, amitriptyline and gabapentin, nortriptyline and propranolol, amitriptyline and propranolol, nortriptyline and verapamil, amitriptyline and verapamil, nortriptyline and acetazolamide, amitriptyline and acetazolamide, paroxetine and topiramate, paroxetine and gabapentin, paroxetine and propranolol, paroxetine and verapamil, paroxetine and acetazolamide, verapamil and topiramate, verapamil and gabapentin, and verapamil and acetazolamide.

Also, in FR-A-2862875 it is disclosed a specific combination treatment of tinnitus, which comprises the administration of a tablet containing ibuprofen (400 mg) at 20:00 hours, a tablet containing ketoprofen (150 mg) at 21:00 hours, and a tablet containing clonazepam (1 mg) and a tablet containing doxylamine succinate (7.5 mg) at 21:30.

Even, the administration of botulinum toxin into the scalp, neck and face muscles, especially in the temporalis muscle, of patients suffering from subjective tinnitus has been disclosed in WO-A-2019/068888.

There are presently no FDA-approved or EMA-approved drugs specifically for tinnitus, and no medications that have been shown to reverse the neural hyperactivity at the root of tinnitus.

According to Shore et al., Maladaptive plasticity in tinnitus-triggers, mechanisms and treatment, Nat. Rev. Neurol., 2016, 12(3), 150-160, many pharmacological agents have been investigated for tinnitus treatment but none has provided convincing reduction of tinnitus impact in excess of placebo effects, and the best-established treatment thus far is cognitive behavioural therapy for which a Cochrane meta-analysis found an improvement in quality of life and reduction of depression scores, even if CBT did not reduce tinnitus loudness or eliminate the percept.

Thus, in view of the large variety of treatments disclosed in the art, which leads to the conclusion that a completely satisfactory treatment of tinnitus is not present, there is still a need to provide an effective treatment of tinnitus, which provides relief from severe tinnitus symptoms, and is able to improve the well-being of the patients.

### Subject-matter of the invention

The subject-matter of the present invention is a ternary combination.

Another aspect of the invention is a composition comprising said ternary combination.

Another aspect of the invention is said ternary combination for use in the treatment of tinnitus and/or hearing loss disorders.

Another aspect of the invention is said ternary combination for use as neuroprotective.

Another aspect of the invention is said composition comprising said ternary combination for use in the treatment of tinnitus and/or hearing loss disorders.

Another aspect of the invention is said composition comprising said combination. for use as neuroprotective.

### Detailed description of the invention

The object of the present invention is a ternary combination consisting essentially of therapeutically effective amounts of nicotinamide, uridine monophosphate and trans-resveratrol.

The inventors of the present invention have developed a ternary combination, which surprisingly is effective in the treatment of all types of tinnitus and/or hearing loss disorders.

In the present description, as well as in the claims, the singular forms "a", "an" and "the" include the plural reference unless the context clearly indicates otherwise. The term "about" refers to a deviation of plus/minus 10%, preferably plus/minus 5%. The percentages are expressed in % by weight (wt%), unless stated the contrary. The ranges defined by the terms "between ... and ..." or by the terms "from ...to..." are meant to include also said stated endpoints thereof, and they also include any narrower sub-range.

The term "therapeutically effective amount" means the amount of a compound that, when administered to a mammal, e.g., a human, for treating tinnitus, is sufficient to effect such treatment for the disease. For the practice of the present invention, it can be determined by one of ordinary skill in the art using known criteria including the age, weight and response of the individual patient, and interpreted within the context of the disease which is being treated or which is being prevented.

### Ternary combination

The ternary combination of the present invention consists essentially of therapeutically effective amounts of nicotinamide, uridine monophosphate and trans-resveratrol.

In a preferred embodiment, the ternary combination of the present invention consists of therapeutically effective amounts of nicotinamide, uridine monophosphate and trans-resveratrol.

The combination of said components is able to ameliorate the symptoms of tinnitus in patients. The presence of the three components is an essential aspect of the invention, as shown in Example 3, wherein the absence of one or two of the components leads to an ineffective composition for treating tinnitus.

Nicotinamide, also named niacinamide or niacin, is a form of vitamin B₃. The structure of nicotinamide consists of a pyridine ring to which a primary amide group is attached in the meta position. It can be prepared from nicotinic acid or nicotinonitrile by known procedures in the art. It is also commercially available through, for example, the company Sigma-Aldrich. In the ternary combination of the invention the content of nicotinamide is comprised between 10 wt% and 65 wt%, preferably between 20 wt% and 55 wt%, preferably between 25 wt% and 50, more preferably between 30 wt% and 45, yet more preferably between 35 wt% and 40 wt%, based on the total weight of the ternary combination. In a preferred embodiment, the content of nicotinamide is between 36 wt% and 38 wt%, based on the total weight of the ternary combination.

Uridine monophosphate, also known as 5'-uridylic acid, is an ester of phosphoric acid with the nucleoside uridine. It consists of the phosphate group, the pentose sugar ribose, and the nucleobase uracil. It may be used as salt, for example, as an alkaline salt, such as monosodium salt or disodium salt. In an embodiment, it is used as disodium salt. It is commercially available through, for example, the company Sigma-Aldrich. In the ternary combination of the invention the content of uridine monophosphate is comprised between 10 wt% and 65 wt%, preferably between 20 wt% and 55 wt%, preferably between 25 wt% and 50, more preferably between 30 wt% and 45, yet more preferably between 35 wt% and 40 wt%, based on the total weight of the ternary combination. In a preferred embodiment, the content of nicotinamide is between 36 wt% and 38 wt%, based on the total weight of the ternary combination.

Trans-resveratrol is a stilbenoid, also named 3,5,4'-trihydroxytrans-stilbene, which is produced by several plants in response to injury or when the plant is under attack by pathogens. It is commercially available through, for example, Sigma-Aldrich. In an embodiment, trans-resveratrol sources for the present invention include, but are not limited to, plant derived extracts, including but not limited to apple extract and grape seed extract. Additionally, non-limiting examples of plants rich in trans-resveratrol suitable for use in the ternary composition of the present invention include: berries (acai, grape, bilberry, blueberry, lingonberry, black currant, chokeberry, blackberry, raspberry, cherry, red currant, cranberry, crowberry, cloudberry, whortleberry, rowanberry), purple corn, purple potato, purple carrot, red sweet potato, red cabbage, eggplant. The resveratrol could be part of a complex mixture obtained by separation technology known in the art aimed at enrichment of the trans-resveratrol and the derivatives or precursors of trans-resveratrol in such mixtures. In the ternary combination of the invention the content of trans-resveratrol is comprised between 5 wt% and 50 wt%, preferably between 10 wt% and 45 wt%, preferably between 15 wt% and 40, more preferably between 20 wt% and 35, yet more preferably between 25 wt% and 30 wt%, based on the total weight of the ternary combination. In a preferred embodiment, the content of trans-resveratrol is between 26 wt% and 28 wt%, based on the total weight of the ternary combination.

### Composition

In an embodiment, the ternary combination is comprised in a composition, which comprises at least a pharmaceutical or nutraceutical acceptable excipient, wherein the components of the ternary combination are the only active ingredients present in the composition.

Therefore, an aspect of the present invention is a composition comprising said ternary combination and at least a pharmaceutical or nutraceutical acceptable excipient, wherein the components of the ternary combination are the only active ingredients present in the composition.

The term "excipient" or "pharmaceutical acceptable excipient" means a component of a pharmaceutic composition that is not an active ingredient, which is useful for preparing the pharmaceutical composition and is generally safe and non-toxic for human use.

The term "excipient" or "nutraceutical acceptable excipient" means a component of a nutraceutical composition that is not an active ingredient, which is useful for preparing the nutraceutical composition and is generally safe and non-toxic for human use.

In the sense of the invention, "nutraceutical composition" means "a functional food composition". Thus, nutraceutical is defined as a food or part of it that provides the body with medical or health benefits, including the prevention and treatment of a disease. It is applied dietary supplements (nutrients), specific diets, and processed foods such as cereals, soups, and beverages that other than nutrition are also used as medicine.

In an embodiment, the composition is a pharmaceutical composition.

The pharmaceutical composition can be prepared using methods that are well known to the skilled in the art such as those contained in handbooks of pharmaceutical technology, such as the book Remington The Science and Practice of Pharmacy, 20th edition, Lippincott, Williams & Wilkins, Philadelphia, 2000 [ISBN: 0-683-306472].

In an embodiment of the invention, the pharmaceutical composition is a composition suitable for oral administration. Any pharmaceutical form suitable for oral administration is included within the scope of the present invention, such as solid compositions in tablet, capsule, powder or granulate form, or liquid compositions, in solution, suspension or syrup form, for example.

In a preferred embodiment, the pharmaceutical composition for oral administration is in solid form, such as, for example, tablets, capsules, powders, granulates; more preferably it is a tablet.

In an embodiment, the composition is a tablet comprising between 5 wt% and 60 wt%, preferably between 10 wt% and 55 wt%, preferably between 15 wt% and 50 wt%, preferably between 20 wt% and 45 wt%, preferably between 25 wt% and 40 wt%, preferably between 30 wt% and 38 wt%, and more preferably between 34 wt% and 37 wt%, on the basis of the total weight of the composition.

In another preferred embodiment, the pharmaceutical composition is in liquid form, such as, for example, syrup, solution, suspension; more preferably it is a syrup.

In an embodiment, the composition is a syrup comprising between 1 wt% and 20 wt%, preferably between 2 wt% and 15 wt%, preferably between 3 wt% and 10 wt%, preferably between 4 wt% and 7 wt%, and more preferably between 5 wt% and 6 wt%, on the basis of the total weight of the composition.

The pharmaceutically acceptable excipients that can be used for preparing pharmaceutical compositions in solid form are well known to those skilled in the art and include, for example, diluents such as calcium carbonate, sodium carbonate, magnesium carbonate, magnesium oxide, calcium sulfate, calcium phosphate, calcium monohydrogen phosphate, sodium chloride, microcrystalline or powdered cellulose, cellulose acetate, ethyl cellulose, dextrates, dextrins, dextrose, lactose, lactitol, fructose, sorbitol, sucrose, maltodextrins, maltose, glyceryl palmitostearate, kaolin, polymethacrylates, pregelatinized starch or starch, among others, and mixtures thereof; lubricants, such as calcium stearate, magnesium stearate, talc, stearic acid, glyceryl behenate, glyceryl palmitostearate, sodium benzoate, sodium lauryl sulfate, sodium stearyl fumarate, or hydrogenated castor oil, among others, and mixtures thereof; disintegrants such as alginic acid, sodium croscarmellose, crospovidone, sodium starch glycolate, starch, low-substituted hydroxypropylcellulose, among others, and mixtures thereof; binding agents such as sodium carboxymethylcellulose, cellulose acetate phthalate, dextrates, dextrin, cyclodextrin, ethylcellulose, guar gum, maltodextrin, methylcellulose, microcrystalline cellulose, povidone, pregelatinized starch, stearic acid, or sucrose, among others, and mixtures thereof; anticaking agents such as tribasic calcium phosphate, calcium silicate, colloidal silica, magnesium silicate, magnesium trisilicate or talc, among others, and mixtures thereof; thickening agents such as colloidal silica, dextrin, ethylcellulose, hydroxyethyl cellulose, hydroxyethylmethyl cellulose, hypromellose, polyethylene glycol, trehalose, xanthan gum, among others, and mixtures thereof; suspending agents such as xanthan gum, guar gum, alginic acid, bentonite, carbomers, sodium or calcium carboxymethylcellulose, hydroxyethyl cellulose, hydroxypropyl cellulose, hydroxypropylmethyl cellulose, hydroxypropyl alginate, microcrystalline or powdered cellulose, anhydrous colloidal silica, dextrins, gelatines, kaolin, magnesium aluminium silicate, maltitol, povidone, sorbitan esters, or tragacanth, among others, and mixtures thereof; stabilizing agents such as guar gum, xanthan gum, alginic acid, ascorbic acid, calcium stearate, sodium carboxymethylcellulose, calcium carboxymethylcellulose, ethylcellulose, lecithin, monoethanolamine, potassium chloride, povidone, sorbitol, or xylitol, among others, and mixtures thereof; flavouring agents such as maltol, vanillin, ethyl vanillin, menthol, citric acid, fumaric acid, ethyl maltol, tartaric acid, peppermint, artificial or natural fruit aromas, among others, and mixtures thereof; sweetening agents such as sorbitol, maltitol, mannitol, dextrose, maltose, xylitol, saccharin, sucrose, sucralose, aspartame, acesulfame potassium, or trehalose, among others, and mixtures thereof; colouring agents such as curcumin, lactoflavin, iron oxides (red, yellow or black), caramel, lactoflavin phosphate, cochineal red, titanium dioxide, or carotenes, among others, and mixtures thereof; or mixtures thereof.

Some of the excipients and carriers suitable to be used in the liquid formulations, in the form of solutions, or suspensions are, for example, solvents such as water, alcohol, almond oil, castor oil, glycerine, PEG, among others; buffering agents such as diethanolamine, dibasic sodium phosphate, monobasic sodium phosphate, potassium citrate, sodium bicarbonate, sodium citrate dihydrate, among others, and mixtures thereof; viscosity modifiers such as alginic acid, bentonite, carbomers, carrageenan, gelatine, glycerine, hydroxyethyl cellulose, hydroxyethylmethyl cellulose, hydroxypropyl cellulose, maltodextrin, polyvinyl alcohol, sodium alginate, tragacanth, Arabic gum, or xanthan gum, among others and mixtures thereof; emulsifying agents such as calcium stearate, cetyl alcohol, ethylene glycol palmitostearate, glyceryl monostearate, lecithin, oleic acid, poloxamers, sodium lauryl sulfate, sorbitan esters, polyoxyethylene castor oil derivatives, or emulsifying wax, among others, and mixtures thereof; suspending agents such as xanthan gum, guar gum, alginic acid, bentonite, carbomers, sodium or calcium carboxymethylcellulose, hydroxyethyl cellulose, hydroxypropyl cellulose, hydroxypropylmethyl cellulose, hydroxypropyl alginate, microcrystalline or powdered cellulose, anhydrous colloidal silica, dextrins, gelatines, kaolin, magnesium aluminium silicate, maltitol, povidone, sorbitan esters, or tragacanth, among others, and mixtures thereof; flocculating agents such as sodium acetate, sodium phosphate, sodium citrate, sodium lauryl sulfate, starch, alginates, tragacanth, or carbomers, among others, and mixtures thereof; wetting agents as benzalkonium chloride, sodium docusate, sodium lauryl sulfate, sorbitan esters, polyoxyethylene stearates or polyoxyethylene sorbitan fatty acid esters, among others, and mixtures thereof; preservatives such as benzalkonium chloride, benzyl alcohol, bronopol, parabens, sodium benzoate, sodium propionate, sodium sorbate, sorbic acid or thimerosal, among others, and mixtures thereof; flavouring agents as maltol, vanillin, ethyl vanillin, menthol, citric acid, fumaric acid, ethyl maltol, tartaric acid, peppermint, artificial or natural fruit aromas, among others, and mixtures thereof; sweetening agents such as sorbitol, maltitol, mannitol, dextrose, maltose, xylitol, saccharin, fructose, sucrose, sucralose, aspartame, acesulfame potassium or trehalose, among others, and mixtures thereof; colouring agents such as curcumin, lactoflavin, iron oxides (red, yellow or black), caramel, lactoflavin phosphate, cochineal red, titanium dioxide, or carotenes, among others, and mixtures thereof; or mixtures thereof.

The excipients suitable to be used in the pharmaceutical compositions of the present invention are well known to those skilled in pharmaceutical technology and are described, for example, in the book R.C. Rowe, P.J. Sheskey and P.J. Weller, *Handbook of Pharmaceutical Excipients,* Fourth Edition, Pharmaceutical Press, 2003.

In an embodiment, the composition is a nutraceutical composition. In a preferred embodiment, the nutraceutical composition is in the form selected from sachets and bars.

In a preferred embodiment, the pharmaceutical or nutraceutical composition comprises, per dose, between 27 mg and 178 mg, preferably between 55 mg and 151 mg, preferably between 69 mg and 137 mg, preferably between 82 mg and 124 mg, and more preferably between 96 mg and 110 mg of nicotinamide; between 14 mg and 138 mg, preferably between 28 mg and 124 mg, preferably between 41 mg and 110 mg, preferably between 55 mg and 96 mg, and more preferably between 69 mg and 83 mg of trans-resveratrol, and between 27 mg and 178 mg, preferably between 55 mg and 151 mg, preferably between 69 mg and 137 mg, preferably between 82 mg and 124 mg, and more preferably between 96 mg and 110 mg of uridine monophosphate.

In a more preferred embodiment, the pharmaceutical or nutraceutical composition comprises, per dose, 100 mg nicotinamide, 75 mg *trans-*resveratrol, and 100 mg uridine monophosphate.

### Treatment of tinnitus and/or hearing loss disorders

An aspect of the invention is the ternary combination for use in the treatment of tinnitus and/or hearing loss disorders.

An aspect of the invention is a composition comprising said ternary combination for use in the treatment of tinnitus and/or hearing loss disorders.

An alternative wording of the use is a method for the treatment of tinnitus and/or hearing loss disorders, which comprises the administration of a therapeutically effective dose of the ternary combination.

An alternative wording of the use is a method for the treatment of tinnitus and/or hearing loss disorders, which comprises the administration of a therapeutically effective dose of the composition comprising said ternary combination.

Surprisingly, the administration of the ternary combination of the invention provides an improvement of the tinnitus, supported both by the audiometry test, and by the subjective perception of the patient.

The assessment of tinnitus may be carried out by means of the visual analogue scale (VAS) to measure the change in tinnitus loudness as perceived at the beginning of the treatment and after the treatment, being the score "10" at the beginning of the treatment, and the score "0" the absence of tinnitus.

The assessment of tinnitus may be carried out also by means of an audiometry test, wherein a pure tone audiometry between 125 and 8000 Hz may be used to assess the auditive status before and after the treatment.

The subjective efficacy perception (tinnitus pitch, distress, and anxiety) by the subject and by the doctor after the treatment may be carried out according to the following score: Bad, Regular, Good, Very Good, and Excellent.

The administration of the composition of the invention provides an improvement in the tinnitus assessment, which is further improved in combination with cochlear electrical stimulation. The use of said electrical stimulation provides an insufficient improvement.

In an embodiment, the use is in combination with cochlear electrical stimulation.

Said electrical stimulation treatment may be carried out according to well-known procedures in the art, for example, as disclosed in Tyler et a/., Electrical Stimulation of the Cochlea to Reduce Tinnitus, Semin. Hear., 2008, 29(4), 326-332, or in Ito et al., Tinnitus suppression by electrical stimulation of the cochlear wall and by cochlear implantation, Laryngoscope, 1994, 104, 752-754.

Audiometry tests reflect an objective improvement in all frequencies in patients with pharmacological treatment, using the ternary combination of the invention, with and without electrical stimulation.

The high efficacy of the treatment of the invention is also reflected by the perception by the subject and by the doctor after the treatment.

Another aspect of the invention is said ternary combination for use as neuroprotective.

Another aspect of the invention is a composition comprising said ternary combination for use as neuroprotective.

An alternative wording of the use is a method for obtaining a neuroprotective effect, which comprises the administration of a therapeutically effective dose of the ternary combination.

An alternative wording of the use is a method for obtaining a neuroprotective effect, which comprises the administration of a therapeutically effective dose of the composition comprising said ternary combination.

The neuroprotective effect of the ternary combination and the composition of the invention is based on: a) the auditive improvement of the audiometry test, which directly measures the neuronal efficiency by transforming a sound wave into an electrical impulse, and b) the disappearance of tinnitus that originates in the brain as a result of the disruption of the signal that reaches the auditory cortex from the cochlea, i.e., the ternary combination of the invention improves the neuronal efficiency of the nervous system.

In a preferred embodiment, the daily dose for use in the treatment of tinnitus and/or hearing loss disorders and for use as neuroprotective is 200 mg nicotinamide, 150 mg trans-resveratrol and 200 mg uridine monophosphate. The daily dose may be administered using two tablets, wherein each one contains 100 mg nicotinamide, 75 mg trans-resveratrol and 100 mg uridine monophosphate.

In an embodiment, the ternary combination for use in the treatment of tinnitus and/or hearing loss disorders and for use as neuroprotective comprises a daily dose comprising 200 mg nicotinamide, 150 mg trans-resveratrol and 200 mg uridine monophosphate.

In an embodiment, the composition for use in the treatment of tinnitus and/or hearing loss disorders and for use as neuroprotective comprises a daily dose comprising 200 mg nicotinamide, 150 mg trans-resveratrol and 200 mg uridine monophosphate.

The present invention may be defined according to the following embodiments:
1.- A ternary combination consisting essentially of therapeutically effective amounts of nicotinamide, uridine monophosphate and trans-resveratrol.
2.- The ternary combination according to embodiment 1, wherein the content of nicotinamide is comprised between 10 wt% and 65 wt%, preferably between 20 wt% and 55 wt%, preferably between 25 wt% and 50, more preferably between 30 wt% and 45, yet more preferably between 35 wt% and 40 wt%, based on the total weight of the ternary combination.
3.- The ternary combination according to embodiment 2, wherein the content of nicotinamide is between 36 wt% and 38 wt%, based on the total weight of the ternary combination.
4.- The ternary combination according to any one of embodiments 1 to 3, wherein the content of uridine monophosphate is comprised between 10 wt% and 65 wt%, preferably between 20 wt% and 55 wt%, preferably between 25 wt% and 50, more preferably between 30 wt% and 45, yet more preferably between 35 wt% and 40 wt%, based on the total weight of the ternary combination.
5.- The ternary combination according to embodiment 4, wherein the content of uridine monophosphate is between 36 wt% and 38 wt%, based on the total weight of the ternary combination.
6.- The ternary combination according to any one of embodiments 1 to 5, wherein uridine monophosphate is used as disodium salt.
7.- The ternary combination according to any one of embodiments 1 to 6, wherein the content of trans-resveratrol is comprised between 5 wt% and 50 wt%, preferably between 10 wt% and 45 wt%, preferably between 15 wt% and 40, more preferably between 20 wt% and 35, yet more preferably between 25 wt% and 30 wt%, based on the total weight of the ternary combination.
8.- The ternary combination according to embodiment 7, wherein the content of trans-resveratrol is between 26 wt% and 28 wt%, based on the total weight of the ternary combination.
9.- A composition comprising the ternary combination according to any one of embodiments 1 to 8 and at least a pharmaceutical or nutraceutical acceptable excipient, wherein the components of the ternary combination are the only active ingredients present in the composition.
10.- The composition according to embodiment 9, wherein it is a pharmaceutical composition.
11.- The composition according to embodiment 10, wherein the composition is suitable for oral administration.
12.- The composition according to embodiment 11, wherein the composition is solid or liquid.
13.- The composition according to embodiment 12, wherein the composition is in solid form selected from tablets, capsules, powders, and granulates.
14.- The composition according to embodiment 13, wherein the composition is a tablet.
15.- The composition according to embodiment 14, wherein the tablet comprises between 5 wt% and 60 wt%, preferably between 10 wt% and 55 wt%, preferably between 15 wt% and 50 wt%, preferably between 20 wt% and 45 wt%, preferably between 25 wt% and 40 wt%, preferably between 30 wt% and 38 wt%, and more preferably between 34 wt% and 37 wt%, on the basis of the total weight of the composition.
16.- The composition according to embodiment 12, wherein the composition is in liquid form selected from syrup, solution, and suspension.
17.- The composition according to embodiment 16, wherein the composition is a syrup.
18.- The composition according to embodiment 17, wherein the syrup comprises between 1 wt% and 20 wt%, preferably between 2 wt% and 15 wt%, preferably between 3 wt% and 10 wt%, preferably between 4 wt% and 7 wt%, and more preferably between 5 wt% and 6 wt%, on the basis of the total weight of the composition.
19.- The composition according to embodiment 9, wherein the composition is a nutraceutical composition.
20.- The composition according to embodiment 19, wherein the composition is in solid form selected from sachets and bars.
21.- The composition according to any one of embodiments 9 to 20, wherein it comprises, per dose, between 27 mg and 178 mg, preferably between 55 mg and 151 mg, preferably between 69 mg and 137 mg, preferably between 82 mg and 124 mg, and more preferably between 96 mg and 110 mg of nicotinamide; between 14 mg and 138 mg, preferably between 28 mg and 124 mg, preferably between 41 mg and 110 mg, preferably between 55 mg and 96 mg, and more preferably between 69 mg and 83 mg of trans-resveratrol, and between 27 mg and 178 mg, preferably between 55 mg and 151 mg, preferably between 69 mg and 137 mg, preferably between 82 mg and 124 mg, and more preferably between 96 mg and 110 mg of uridine monophosphate.
22.- The composition according to embodiment 21, wherein the composition comprises, per dose, 100 mg nicotinamide, 75 mg trans-resveratrol, and 100 mg uridine monophosphate.
23.- The ternary combination according to any one of embodiments 1 to 8 for use in the treatment of tinnitus and/or hearing loss disorders.
24.- The ternary combination for use according to embodiment 23, wherein it is administered in combination with cochlear electrical stimulation.
25.- The composition according to any one of embodiments 9 to 22 for use in the treatment of tinnitus and/or hearing loss disorders.
26.- The composition for use according to embodiment 25, wherein it is administered in combination with cochlear electrical stimulation.
27.- The ternary combination according to any one of embodiments 1 to 8 for use as neuroprotective.
28.- The composition according to any one of embodiments 9 to 22 for use as neuroprotective.
29.- The ternary combination for use according to any one of embodiments 23, 24 or 27, wherein the daily dose is 200 mg nicotinamide, 150 mg trans-resveratrol and 200 mg uridine monophosphate.
30.- The composition for use according to any one of embodiments 25, 26 or 28, wherein the daily dose is 200 mg nicotinamide, 150 mg trans-resveratrol and 200 mg uridine monophosphate.

### Examples

### Example 1: Tablet

Tablets were prepared using well-known procedures in the art, which comprised the following components per tablet as shown in Table I:

**TABLE I**

| Component | mg |
|---|---|
| Nicotinamide | 100 |
| Uridine monophosphate disodium salt | 112.50 |
| *trans*-resveratrol | 75 |
| γ-cyclodextrin | 100 |
| Sodium carboxymethylcellulose | 20 |
| Calcium monohydrogen phosphate dihydrate | 152.20 |
| Microcrystalline cellulose | 212.30 |
| Magnesium stearate | 8 |
| Micronized talc | 16 |
| Silicon dioxide | 4 |

### Example 2: Syrup

A syrup was prepared using well-known procedures in the art, which comprised the following components as shown in Table II:

**TABLE II**

| Component | Amount |
|---|---|
| Water | 400 mL |
| Fructose | 500 mg |
| Nicotinamide | 10 g |
| Uridine monophosphate disodium salt | 10 g |
| *trans*-resveratrol | 7.5 g |
| Sodium citrate | 100 mg |
| Sodium carboxymethylcellulose | 20 |
| Potassium sorbate | 120 mg |
| PEG 400 | 80 ml |
| Sodium hydroxide | q.s. pH 5 |

### Example 3: Clinical case

Treatment of a subject with tinnitus was carried out by administering the following compositions according to the regime shown in Table III. The result after the treatment is expressed according to the visual analogue scale, wherein the score "0" refers to absence of tinnitus and the score "10" is tinnitus at time t=0 of the treatment.

**TABLE III**

| Composition | Amount | Duration | Result at the end of the treatment |
|---|---|---|---|
| UMP | 200 mg/day | 60 days | 10 |
| *trans*-resveratrol | 150 mg/day | 60 days | 10 |
| Nicotinamide | 200 mg/day | 60 days | 10 |
| Nicotinamide *trans*-resveratrol | 200 mg/day | 60 days 10 | |
| | 150 mg/day | | |
| Nicotinamide | 200 mg/day | 60 days | 10 |
| UMP | 200 mg/day | | |
| UMP | 200 mg/day | 60 days | 10 |
| *trans*-resveratrol | 150 mg/day | | |
| Nicotinamide | 200 mg/day | 60 days | 7 |
| *trans*-resveratrol | 150 mg/day | | |
| UMP | 200 mg/day | | |

After finishing each treatment there was a lapse of 30 days to avoid any interference between treatments.

It was observed that only the use of the three components of the ternary combination of the present invention, there was an improvement in the tinnitus assessment.

A treatment with cochlear electrical stimulation, twice a week during 8 weeks produced also an improvement in the tinnitus assessment, with a score of 7.

The combination of electrical stimulation and the ternary combination produced the best result in the tinnitus assessment, with a score of 5.

### Example 4: Clinical study

The primary objective of this study was to assess the efficacy of the ternary combination of the invention in ameliorating tinnitus symptoms in afflicted patients.

A group of 26 subjects, aged 27 - 74, was treated for a period of 30 days, with 2 tablets/day, each tablet comprising 100 mg of nicotinamide, 112.5 mg of uridine monophosphate disodium salt (equivalent to 100 mg of uridine monophosphate), and 75 mg of *trans*-resveratrol.

A subgroup of 14 subjects was treated simultaneously with the pharmacological treatment and cochlear electrical stimulation.

The inclusion criteria for participating in the clinical study were:
- male or female subjects with a persistent, subjective, uni or bi-lateral tinnitus
- duration of tinnitus was greater than 6 months

After the treatment, three different responses were assessed:
a) The assessment of tinnitus was carried out by means of the visual analogue scale (VAS) to measure the change in tinnitus loudness as perceived at the beginning of the treatment and after the treatment, being the score "10" at the beginning of the treatment, and the score "0" the absence of tinnitus.
b) A pure tone audiometry between 125 and 8000 Hz was carried out before and after the treatment.
c) Efficacy perception (tinnitus pitch, distress, and anxiety) by the subject and by the doctor after the treatment according to the following score: Bad, Regular, Good, Very Good, and Excellent.

The results regarding the assessment of tinnitus showed a significant reduction of the tinnitus both in the group without electrical stimulation (9 subjects) and in the group with electrical stimulation (4 subjects), being the reduction in the latter case significantly better: the score was "3.5" for the treatment comprising the pharmacological treatment and the electrical stimulation, and the score was "4.9" for the pharmacological treatment without electrical stimulation.

The audiometry reflected an improvement in all frequencies (expressed as Delta dB) in both treatment groups, i.e., pharmacological treatment with and without electrical stimulation (ES), as shown in Table IV:

**TABLE IV**

| Frequency (Hz) | Delta dB Treatment without ES | Delta dB Treatment with ES |
|---|---|---|
| 125 | -7 | -48 |
| 250 | -10 | -50 |
| 500 | -8 | -43 |
| 1000 | -6 | -55 |
| 2000 | -5 | -43 |
| 4000 | -2 | -33 |
| 8000 | -6 | -35 |

Perception by the subject and by the doctor after the treatment reflected a high efficacy of the treatment, wherein it was scored as Very Good or Excellent in all de cases, as shown in Table V:

**TABLE V**

| Score | Doctor | Subject |
|---|---|---|
| Very Good | 23 | 27 |
| Excellent | 77 | 73 |

The perception of the doctor is a direct correlation of the audiometry and the perception of the subject correlates with the life quality.

It can be observed that the ternary combination of the present invention is highly effective in the treatment of tinnitus as well as in the treatment of hearing loss disorders.

## Claims

1. A ternary combination consisting essentially of therapeutically effective amounts of nicotinamide, uridine monophosphate and trans-resveratrol.

2. The ternary combination according to claim 1, wherein the content of nicotinamide is comprised between 10 wt% and 65 wt%, preferably between 20 wt% and 55 wt%, preferably between 25 wt% and 50, more preferably between 30 wt% and 45, yet more preferably between 35 wt% and 40 wt%, based on the total weight of the ternary combination.

3. The ternary combination according to claim 1 or 2, wherein the content of uridine monophosphate is comprised between 10 wt% and 65 wt%, preferably between 20 wt% and 55 wt%, preferably between 25 wt% and 50, more preferably between 30 wt% and 45, yet more preferably between 35 wt% and 40 wt%, based on the total weight of the ternary combination.

4. The ternary combination according to any one of claims 1 to 3, wherein the content of trans-resveratrol is comprised between 5 wt% and 50 wt%, preferably between 10 wt% and 45 wt%, preferably between 15 wt% and 40, more preferably between 20 wt% and 35, yet more preferably between 25 wt% and 30 wt%, based on the total weight of the ternary combination.

5. A composition comprising the ternary combination according to any one of claims 1 to 4 and at least a pharmaceutical or nutraceutical acceptable excipient, wherein the components of the ternary combination are the only active ingredients present in the composition.

6. The composition according to claim 5, wherein it is a pharmaceutical composition.

7. The composition according to claim 6, wherein the composition is suitable for oral administration.

8. The composition according to claim 7, wherein the composition is solid or liquid.

9. The composition according to claim 8, wherein the composition is in solid form selected from tablets, capsules, powders, and granulates.

10. The composition according to claim 9, wherein the composition is a tablet.

11. The composition according to claim 5, wherein the composition is a nutraceutical composition.

12. The composition according to any one of claims 5 to 11, wherein it comprises, per dose, between 27 mg and 178 mg, preferably between 55 mg and 151 mg, preferably between 69 mg and 137 mg, preferably between 82 mg and 124 mg, and more preferably between 96 mg and 110 mg of nicotinamide; between 14 mg and 138 mg, preferably between 28 mg and 124 mg, preferably between 41 mg and 110 mg, preferably between 55 mg and 96 mg, and more preferably between 69 mg and 83 mg of trans-resveratrol, and between 27 mg and 178 mg, preferably between 55 mg and 151 mg, preferably between 69 mg and 137 mg, preferably between 82 mg and 124 mg, and more preferably between 96 mg and 110 mg of uridine monophosphate.

13. The ternary combination according to any one of claims 1 to 4 or the composition according to any one of claims 5 to 12 for use in the treatment of tinnitus and/or hearing loss disorders.

14. The ternary combination for use or the composition for use according to claim 13, wherein it is administered in combination with cochlear electrical stimulation.

15. The ternary combination according to any one of claims 1 to 4 or the composition according to any one of claims 5 to 12 for use as neuroprotective.
